# EUROPEAN PATENT SPECIFICATION

(11) **EP 0 540 782 B1**
(45) Date of publication and mention of the grant of the patent: **06.05.1999**
(21) Application number: 91202616.8
(22) Date of filing: 07.10.1991
(51) Int. Cl.: A61K 31/66

(54) **Use of analogues of polyisoprenyl pyrophosphate for inhibiting protein isoprenylation**
Verwendung von Polyisoprenyl pyrophosphate Analogen zur Hemmung des Proteinisoprenylations
Utilisation d'analogues de polyisoprenyl pyrophosphate pour inhiber l'isopenylation de protéine

(43) Date of publication of application: 12.05.1993
(73) Proprietor: NEDERLANDSE ORGANISATIE VOOR TOEGEPAST-NATUURWETENSCHAPPELIJK ONDERZOEK TNO, 2628 VK Delft (NL); Rijksuniversiteit te Leiden, 2300 RA Leiden (NL)
(72) Inventor: Cohen, Louis H., NL-3621 HR Breukelen (NL); Van Boom, Jacobus H., NL-2253 RD Voorschoten (NL); Van der Marel, Gijsbert A., NL-2318 MD Leiden (NL); Valentijn, Adrianus P. R. M., NL- Leiden (NL)
(74) Representative: de Bruijn, Leendert C.

(56) References cited:
- EP-A- 356 866
- EP-A- 0 324 421
- EP-A- 0 350 801
- EP-A- 0 418 814
- BIOCHEMICAL AND BIOPHYSICAL RESEARCH COMMUNICATIONS, vol. 181, no. 2, 1991, pages 729-735; P.D. NAGARATNAM et al.: "Inhibition of farnesyl transferases from malignant ans non-malignant cultured human lymphocytes by prenyl substrate analogues"
- CHEMICAL ABSTRACTS, vol. 114, no. 15, 15th April 1991, page 29, abstract no. 135661j, Columbus, Ohio, US; V.V. FEDUROV et al.: "Cytotoxic effects of amino and phosphonate analogs of natural prenylpyrophosphates", & DOKL. AKAD. NAUK. UKR. SSR, SER. B: GEOL., KHIM. BIOL. NAUKI 1990, (9), 79-82
- SYNLETT, no. 9, September 1991, pages 663-664; A.R.P.M. VALENTIJN et al.: "An expeditious synthesis of pyrophosphate analogues of farnesyl pyrophosphate using the phosphonylating agent methyl methylphosphonomorpholidate"
- JOURNAL OF BIOLOGICAL CHEMISTRY, vol. 265, no. 25, 1990, pages 14701-14704; M.D. SCHABER et al.: "Polyisoprenylation of Ras in vitro by a farnesyl-protein transferase"
- SCIENCE, vol. 245, 1989, pages 379-385; W.R. SCHAFER et al.: "Genetic and pharmacological suppression of oncogenic mutations in RAS genes of yeast and humans"

## Description

The present invention is in the field of medicine. More specifically, it relates to a novel use of polyisoprenyl pyrophosphate analogues in interfering with certain biochemical processes. In particular analogues wherein the polyisoprenyl group is farnesyl or geranylgeranyl.

Recently the post-translational modification of mammalian cell proteins involving thioether derivatisation of carboxyl-terminal cysteine residues by mevalonate-derived isoprenyl groups was discovered. Several of these isoprenylated proteins were identified as belonging to groups of related proteins: e.g. the nuclear lamins, low molecular weight GTP binding proteins, such as the ras-oncogene proteins and heteromeric G proteins (Schäfer et al., *Science* **245** (1989) 379-385). The polyisoprenyl group that was attached to a protein was identified as either farnesyl (C₁₅) or geranylgeranyl (C₂₀), probably depending on the recognition of the C-terminal amino acid sequence of the proteins involved. Lamins and p21^{ras} proteins, which possess the consensus CAAX motif (C = cysteine, A = any amino acid having an aliphatic side chain and X = any amino acid), are farnesylated, while several members of the rab proteins having C-terminal CC/CXC motifs, and of the heteromeric G-protein γ-subunits are geranylgeranylated.

The isoprenylation of these proteins seems to play a role in their association with membranes and nuclear envelopes, where they are processed further and/or perform their function. This was shown for example by blocking the mevalonate synthesis by HMG-CoA reductase inhibitors, which prevented proteolytic processing of the lamin A precursor (Beck et al., *J. Cell. Biol.* **110** (1990) 1489-1499) or resulted, in other studies, in the accumulation of non-isoprenylated p21^{ras} precursor and the loss of transforming activity of oncogenic ras proteins. A review of the post-translational modification of proteins by isoprenoids in mammalian cells is given by Maltese W.A. in *FASEB* J. **4** 3319-3329 (1990). The latter observation triggered the search for specific inhibitors of the farnesylation of p21^{ras} in order to prevent its action in cells, where overexpression of this protein leads to tumour development, such as in colon carcinomas.

G proteins play a role in the receptor-mediated transduction of signals (such as growth modulation signals) over the plasma membrane, and other isoprenylated proteins, not yet identified, may have a function in cell cycle progression. There is some evidence as well that GTP binding proteins are involved in the regulation of intracellular protein traffic and secretion. There is even some suggestion that isoprenylated proteins play a role in the translational control of HMG-CoA reductase, the rate limiting enzyme of the isoprene and subsequent cholesterol synthesis.

The enzymes involved in the protein isoprenylation process, isoprenyl: protein transferases, are reported to use *all*-*trans*farnesyl-pyrophosphate (FPP) as a substrate for the addition of the farnesyl group to the protein. FPP may also be a substrate in the production of geranylgeranyl pyrophosphate, which is subsequently used in the synthesis of geranylgeranylated proteins.

It is known that farnesyl pyrophosphate is a substrate in a key step in the synthesis of cholesterol. In this step the synthesis of squalene from farnesyl pyrophosphate is effected by the enzyme squalene synthase. In this step squalene is formed by formation of a bond between two farnesyl groups in a reaction that requires a synthase. In EP-A-324421 and EP-A-356886 certain farnesyl pyrophosphate analogues have been described that can be used for inhibiting said step in the cholesterol synthesis, for use in the treatment of hypercholesterolaemia. Such a process differs from the protein isoprenylation in which other enzymes are required for the isoprenylation, e.g. the farnesylation, or the geranylgeranylation of proteins.

It is an object of the present invention to provide means for inhibiting protein isoprenylation, in order to prevent or inhibit undesired biological processes such as tumour development.

This object is achieved by using a polyisoprenyl pyrophosphate analogue for preparing a pharmaceutical composition suitable for inhibiting protein isoprenylation,
the specific polyisoprenyl pyrophosphate analogues having formula 1, 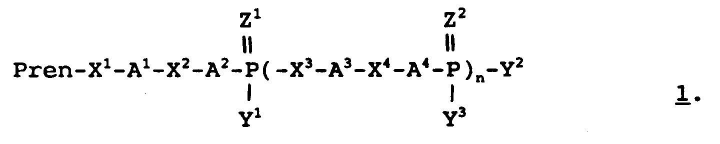 wherein:
Pren represents a C₁₀-C₃₀ terpenoid group or a derivative thereof;
A¹, A², A³ and A⁴ independently represent a direct bond or a C₁-C₄ alkylene or alkenylene group, optionally having substituents selected from methyl, hydroxy, methoxy, mercapto, methylthio, amino, methylamino, dimethylamino and halogen;
X¹, X², X³ and X⁴ independently represent a direct bond, oxygen, sulphur, imino or methylimino, with the proviso that if A¹ represents a direct bond, X² also represents a direct bond and vice versa, and if A³ represents a direct bond, X⁴ also represents a direct bond and vice versa;
Y¹, Y² and Y³ independently represent hydroxy, alkoxy, mercapto, alkylthio, amino, mono- or di-alkylamino, alkyl, alkenyl, alkynyl, cycloalkyl, aryl or arylalkyl, wherein alkyl, alkoxy, alkenyl and alkynyl are linear or branched having 1-6 carbon atoms and may have substituents selected from hydroxy, methoxy, mercapto, methylthio, amino, methylamino, dimethylamino and halogen, cycloalkyl having 3-8 carbon atoms, aryl is carbocyclic or heterocyclic having 5-10 ring atoms and cycloalkyl and aryl may have substituents selected from methyl, hydroxy, methoxy and halogen, whereby one of Y¹, Y² and Y³ may also represent a C₇-C₃₀ alkyl or alkenyl group, including a C₁₀-C₃₀ terpenoid group;
Z¹ and Z² independently represent oxygen or sulphur; and
n is 1 or 2;
or a pharmaceutically acceptable salt thereof;
with the proviso that the polyisoprenyl pyrophosphate analogue is not the polyisoprenyl pyrophosphate itself.

The present invention further relates to a pharmaceutical composition for inhibiting protein isoprenylation, containing a polyisoprenyl pyrophosphate analogue according to formula 1, wherein X³-A³-X⁴-A⁴ represents an oxygen atom or a methyleneoxy group, or a pharmaceutically acceptable salt thereof, with the proviso that the polyisoprenyl pyrophosphate analogue is not polyisoprenyl pyrophosphate itself, together with a carrier adapted for protein isoprenylation inhibiting purposes.

In the above formula 1, the group Pren generally represents an acyclic terpenoid group, i.e. a hydrocarbon group consisting of C₅ isoprenylene (2-methyl-1,4-but-2-enylene) units, in particular 2-6 of such isoprenylene units. One or more of these isoprenylene units may be replaced by isomeric units such as 2-methyl-1,2-but-3-enylene or 2-methylene-1,4-butylene. The C-C double bond in these units may be *cis* and/or *trans*, but is preferably *trans*. One or more of the double bonds may be hydrogenated resulting in a terpenoid group having less than one double bond per C₅ moiety, or alternatively one or more of the single C-C bonds may be dehydrogenated resulting in a terpenoid group having more than one double bond per C₅ moiety. Preferably the terpenoid group contains one C-C double bond per C₅ moiety. The terpenoid group may be substituted e.g. by halogen, in particular fluorine, methoxy or additional methyl; on the other hand one or more methyl side groups may be lacking. Preferably the terpenoid group is a C₁₀-C₂₀ group (monoterpenic, sesquiterpenic or diterpenic). Examples of monoterpenic groups (C₁₀) are geranyl, neryl, linalyl, ocimenyl, myrcenyl and citronellyl. Examples of sesquiterpenic groups are α-farnesyl, β-farnesyl, nerolidyl and dihydro derivatives thereof. Examples of diterpenic groups are geranyl-geranyl, geranyl-linalyl and hydrogenated derivatives thereof such as geranyl-citronellyl and phytyl. Preferred terpenoid groups are farnesyl and geranyl-geranyl. Most preferred is *all*-*trans*-α-polyisoprenyl.

The two groups represented by X¹-A¹-X²-A² and X³-A³-X⁴-A⁴ in formula 1 are in particular each a direct bond between the terpenoid group and the phosphorus atom or a heteroatom such as oxygen, nitrogen or sulphur, or an alkylene group, or an alkylene group flanked by one or two heteroatoms or a heteroatom flanked by two alkylene groups. The heteroatom is preferably oxygen or sulphur, most preferably oxygen. The alkylene group may have substituents such as fluorine, chlorine, methyl, hydroxy or methoxy, and it is preferably methylene. The group X¹-A¹-X²-A² is more in particular a direct bond, a methylene group, a methyleneoxy group or a substituted methylene of methyleneoxy group. The group X³-A³-X⁴-A⁴ is more in particular an oxygen atom, a methylene group, a methyleneoxy group or a substituted methylene or methyleneoxy group. The multiplicator n may have a value of 1 or 2, representing for example pyrophosphates (diphosphonates), and triphosphates respectively, but is preferably 1.

The groups Y¹, Y² and Y³ in formula 1 preferably comprise one or more hydrophilic groups, such as hydroxy, hydroxyethyl, mercapto or amino. In particular, at least one of Y¹, Y² and Y³, and more in particular at least two of Y¹, Y² and Y³ are hydroxy, mercapto or salts thereof; the remaining ones may for example be methyl, ethyl, phenyl, benzyl, methoxy, farnesyloxy, phenoxy, cyclohexyloxy, methylthio, dimethylamino etc. Also, Y¹ and Y² or Y¹ and Y³ may be connected to each other, e.g. as a cyclic phosphoric diester or a cyclic phosphonate. Where any of Y¹, Y² and Y³ represents hydroxy or mercapto, the resulting phosphoric, phosphonic or phosphinic acid may be in the form of a pharmaceutically acceptable salt, e.g. an ammonium, substituted ammonium, alkali metal or alkaline earth metal salt. Preferred compounds are those wherein Y¹, Y² and Y³ each represent hydroxy or mercapto, preferably hydroxy, and in particular the salts thereof.

The groups Z¹ and Z² in formula 1 are oxygen or sulphur, preferably oxygen.

Examples of poylisoprenyl pyrophosphate analogues to be used according to the invention include farnesylmethylphosphonophosphate (1), farnesylmethylphosphono-methylphosphonate (2), farnesylphosphonophosphate (3), farnesylphosphono-methylphosphonate (4) and farnesylthiophosphonophosphate and the corresponding geranylgeranyl compounds, with a preference for compounds (1) and (3).

The polyisoprenyl pyrophosphate analogues to be used according to the invention can be prepared in a manner which is known per se. For example, they may be prepared by the method described by Valentijn et al, *Synlett*. **1991**, 663-664, or by the methods described in European patent applications 324421 and 356866.

Surprisingly it was found that the polyisoprenyl pyrophosphate analogues according to the invention are capable to specifically inhibit the protein isoprenylation at low dosage amounts (IC₅₀ < 1 µM) and that squalene synthesis is not inhibited significantly at these dosage amounts. Preferably the FPP analogue concentration will be selected in order to disturb other processes which use FPP or similar substrates, e.g. the squalene synthesis, as little as possible. Therefore, preferred FPP analogues to be used according to the invention are analogues that are specific for the isoprenylation reaction to be inhibited.

For example the compounds (1) and (3) mentioned above can inhibit p21^{ras}: farnesyl transferase when applied in dosage amounts that are too small for said analogues to inhibit squalene synthase, thereby inhibiting farnesylation of p21^{ras} without inhibiting squalene synthesis.

The polyisoprenyl pyrophosphate analogues described above are useful as an active substance in a pharmaceutical composition intended to interfere with protein isoprenylation. As such, they are useful as inhibitors in processes such as oncogenesis and other unwanted cell proliferation, and furthermore as suppressants of aberrant high signal transduction.

The pharmaceutical compositions to be prepared using the polyisoprenyl pyrophosphate analognes according to the invention may be formulated in a usual way, e.g. by associating the polyisoprenyl pyrophosphate analogue with a suitable solid or liquid carrier and optional adjuvants or other active components. The composition may be suitable for oral administration (capsule, pill, tablet, gel, powder, sachet, syrup, solution, dispersion etc.) or may be an injectable solution or another administration form. The composition may be administered to mammalians including man, in a dose which depends on the particular purpose of the administration, the body weight and other conditions well known to the skilled person. A dose can be administered in a single dosage or in several daily dosages.

### Example I

### Preparation of farnesylmethylphosphonophosphate (1).

### Methyl methylphosphonomorpholidate (a).

Methyl phosphonic dichloride (6.65 g, 50 mmol) as dissolved in freshly distilled ether (80 ml) and cooled to 0°C. Then morpholine (8.72 ml, 100 mmol) in 20 ml ether was added dropwise over a period of 2 h and stirring was continued overnight at 0°C. The salts were removed by filtration and the solvent was evaporated. The residue was dissolved in 50 ml ether and treated with a mixture of methanol (3.82 ml, 100 mmol) and triethylamine (6.97 ml, 50 mmol) in 50 ml ether at 0°C. After overnight stirring at 0°C the salts were removed by filtration, followed by concentration. Compound (a) was purified by distillation (bp. 118°C at 0.7 mm Hg) and obtained in a yield of 79%.
¹³C NMR (CDCl₃) δ (ppm) 7.9, 10.8 (CH₃, J_{CP} = 144.9 Hz); 43.4 (CH₂); 51.6, 51.7 (OCH₃); 66.7 (CH₂).
³¹P NMR (CDCl₃) δ 32.8

### Farnesyl chloride (b)

N-Chlorosuccinimide (0.66 g, 4.95 mmol) was dissolved in 20 ml of dry CH₂Cl₂ under a nitrogen atmosphere. The solution was cooled to -30°C and 0.37 ml dimethyl sulphide (5 mmol) was added. The mixture was allowed to warm to 0°C before it was cooled to -40°C. Then farnesol (1 g, 4.5 mmol) dissolved in 2.5 ml CH₂Cl₂ was added dropwise to the mixture over a period of 3 minutes. The reaction mixture was warmed to 0°C in 1 h, at which temperature it was maintained for another hour. After 15 minutes of stirring at room temperature the reaction mixture was poured into a separatory funnel which contained 12.5 ml of cold saturated NaCl. The aqueous layer was extracted with two 10 ml portions of pentane. The organic layers were combined with an additional 10 ml of pentane and washed with two 5 ml portions of cold saturated NaCl. The organic layer was dried over MgSO₄ and concentrated to dryness *in vacuo*. Compound (b) was thus obtained in a yield of 89%.
¹³C NMR (CDCl₃) δ 16.0, 17.6, 25.7 (4 x CH₃); 26.1, 26.7, 39.4, 39.7 (C4, C5, C7, C8); 41.1 (C1); 120.3, 124.0, 124.3 (C2, C6, C10); 130.3, 135.5 (C3, C7, C9).
¹H NMR (CDCl₃) δ 1.60 (s, 6H, 2 x CH₃); 1.68 (s, 3H, CH₃); 2.01-2.35 (m, 8H, H₄, H₅, H₈, H₉); 4.07, 4.11 J_{1,2} = 8 Hz (d, 2H, H₁); 7.15-7.29 (m, 3H, H₂, H₆, H₁₀)
Rf: 0.64 (ether/light petroleum 1/1 v/v).

### Farnesylmethyl-methylphosphonomorpholidate (c).

Compound (a) (376 mg 2.1 mmol) was dissolved in 10 ml freshly distilled THF and cooled to -78°C under an argon atmosphere. Then 1.3 ml n-butyllithium (1.6 M in hexane, 2.1 mmol) was added and stirring was continued for 20 minutes, followed by dropwise addition of compound (b) (481 mg, 2 mmol) in 5 ml THF over a period of 5 minutes. After additional stirring for 1 h at -78°C, work-up and chromatographical purification, the desired compound (c) was obtained as a colourless oil in a yield of 63%.
¹³C NMR (CDCl₃) δ 15.9, 17.5, 25.6 (4 x CH₃); 20.7, 20.8 (C2); 23.4, 26.1 (C1); 26.3, 26.6 (C5, C9); 39.4, 39.6 (C5, C10); 52.0, 52.1 (2 x OCH₃); 122.6, 122.9 (C3); 123.8, 124.9 (C7,C11); 131.0, 134.9, 136.4 (C4, C8, C12).
¹H NMR (CDCl₃) δ 1.58 (s, 6H, 2 x CH₃), 1.62 (s, 3H, CH₃), 1.68 (s, 3H, CH₃), 1.71-1.82 (m, 2H, H₁); 1.97-2.09 (m, 8H, H₅, H₈, H₉, H₁₀); 2.23-2.34 (m, 2H, H₃); 3.72, 3,76 (2 x s, 6H, 2 x OCH₃); 5.07-5.30 (m, 3H, H₃, H₇, H₁₂).
³¹P NMR (CDCl₃) δ 33.0.
Rf 0.17 (5% acetone in dichloromethane)

### Farnesylmethylphosphonomorpholidate (d)

Compound (c) (383 mg, 1 mmol) was coevaporated three times with toluene and dissolved in 2 ml dry acetonitrile. Then trimethylsilyl bromide (1.1 mmol) was added. As ³¹P NMR (δ 27.9) showed that the reaction had gone to completion the reaction mixture was concentrated and treated with 1 ml of a 1M solution of tetrabutylammonium fluoride in dry dioxane. Again ³¹P NMR showed that the reaction was complete (δ 20.0), and compound (d) which was obtained was not isolated, but used in the next step without purification.

### Farnesylmethylphosphonophosphate (1).

Compound (d) was dissolved in 3 ml dry pyridine and 3 mmol tributylammonium phosphonic acid was added. Following evaporation of the solvent, the mixture was redissolved in dry pyridine and stirred for two days at room temperature. Then the reaction mixture was concentrated to dryness, dissolved in a small amount of 2-propanol/0.15 M NH₄HCO₃ (1/49 v/v) and applied to a Dowex 50W x 4 cation exchange resin (NH₄⁺ form). After elution with the same solvent the eluate was concentrated to dryness. Purification was accomplished by gelfiltration on a High Load 26/60 Sephacryl S-100 High Resolution column, eluting with a solution of 30% methanol in 0.15 M NH₄HCO₃. After lyophilization of the appropriate fractions compound (1) was obtained as a white powder in a yield of 65%.
¹H NMR (CD₃OD) δ 1.59 (s, 6H, 2 x CH₂); 1.65, 1,66 (2x, s, 6H, 2x CH₃); 1.99-2.16 (m, 10H, H₁, H₅, H₈, H₉, H₁₀); 2.28-2.39 (m, 2H, H₂); 5.17-5.29 (m, 3H, H₃, H₇, H₁₂).
³¹P NMR (D₂O) δ -9.8, -9.5, 16.4, 16.7 (J_{PP} = 26.3 Hz).

### Example 2

The inhibition of the farnesylation of p21^{ras} precursor by the enzyme farnesyl: protein transferase present in rabbit reticulocyte lysate by farnesylmethylphosphonophosphate (compound 1) and farnesylphosphonophosphate (compound 3) was tested. The prenylation of bacterially expressed, unprocessed H-ras protein (30 µg/ml) was determined in untreated reticulocyte lysate (Promega) using 1 µM of [³H]-farnesyl pyrophosphate (ARC Inc.; 15 Ci/mmol), 1 mM MgCl₂ and 1 mM DTT. The incubations were conducted at 37°C for 30 min in the presence of different amounts of compound 1 or 3. Final incubation volume was 25 µl. The incorporation of [³H]- FPP into TCA precipitable material was determined as described by Reiss et al., Cell 62 (1990) 81-88. The difference between the values obtained in the presence of ras-protein and those obtained without this addition (blank) was a measure for the remaining enzyme activity. This activity was concentration-dependently decreased by both compounds. From the dose-response curve the IC₅₀-values were calculated, which were 4 µM and < 1 µM for compound 1 and 3, respectively.

Similarly the IC₅₀-values were obtained for the compounds 1 and 3 respectively for inhibition of the enzyme squalene synthase in rat liver microsomal preparations. The IC₅₀-values were 480 µM and 120 µM respectively.

## Claims

1. Use of a polyisoprenyl pyrophosphate analogue for preparing a pharmaceutical composition suitable for inhibiting protein isoprenylation, the polyisoprenyl pyrophosphate analogue having formula 1, 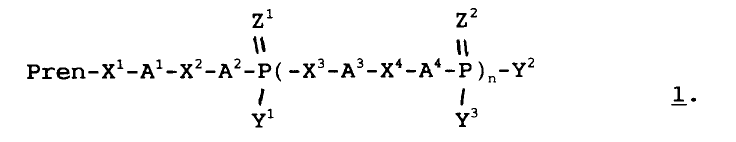 wherein:
Pren represents a C₁₀-C₃₀ terpenoid group or a derivative thereof;
A¹, A², A³ and A⁴ independently represent a direct bond or a C₁-C₄ alkylene or alkenylene group, optionally having substituents selected from methyl, hydroxy, methoxy, mercapto, methylthio, amino, methylamino, dimethylamino and halogen;
X¹, X², X³ and X⁴ independently represent a direct bond, oxygen, sulphur, imino or methylimino, with the proviso that if A¹ represents a direct bond, X² also represents a direct bond and vice versa, and if A³ represents a direct bond, X⁴ also represents a direct bond and vice versa;
Y¹, Y² and Y³ independently represent hydroxy, alkoxy, mercapto, alkylthio, amino, mono- or di-alkylamino, alkyl, alkenyl, alkynyl, cycloalkyl, aryl or arylalkyl, wherein alkyl, alkoxy, alkenyl and alkynyl are linear or branched having 1-6 carbon atoms and may have substituents selected from hydroxy, methoxy, mercapto, methylthio, amino, methylamino, dimethylamino and halogen, cycloalkyl has 3-8 carbon atoms, aryl is carbocyclic or heterocyclic having 5-10 ring atoms and cycloalkyl and aryl may have substituents selected from methyl, hydroxy, methoxy and halogen, whereby one of Y¹, Y² and Y³ may also represent a C₇-C₃₀ alkyl or alkenyl group, including a C₁₀-C₃₀ terpenoid group;
Z¹ and Z² independently represent oxygen or sulphur; and
n is 1 or 2;
or a pharmaceutically acceptable salt thereof;
with the proviso that the polyisoprenyl pyrophosphate analogue is not polyisoprenyl pyrophosphate itself.

2. Use according to claim 1, wherein the group X³-A³-X⁴-A⁴ represents an oxygen atom or a methyleneoxy group.

3. Use according to claim 1 or 2, wherein: Pren represents a farnesyl or geranyl-geranyl group.

4. Use according to any one of claims 1-3, wherein: A¹ and A³ independently represent a direct bond or a methylene group and at least two of Y¹, Y² and Y³ are hydroxy or mercapto or a salt thereof; and n = 1.

5. Use according to any of claims 1-4, wherein: Y¹, Y² and Y³ are hydroxy or mercapto or a salt thereof.

6. Use according to any of the previous claims, wherein the pharmaceutical composition is suitable for the prophylaxis and/or treatment of carcinomas.

7. Use according to any of the previous claims, wherein the pharmaceutical composition is suitable for the inhibition of the isoprenylation of a ras protein.

8. Pharmaceutical composition suitable for inhibiting protein isoprenylation, containing a polyisoprenyl pyrophosphate analogue having formula 1: 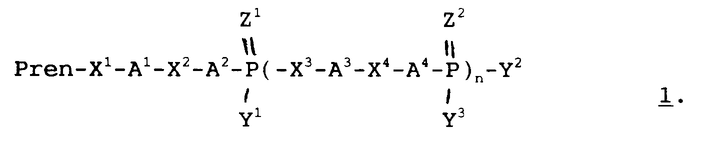 wherein:
Pren represents a C₁₀-C₃₀ terpenoid group or a derivative thereof;
A¹ and A² independently represent a direct bond or a C₁-C₄ alkylene or alkenylene group, optionally having substituents selected from methyl, hydroxy, methoxy, mercapto, methylthio, amino, methylamino, dimethylamino and halogen;
X¹ and X² independently represent a direct bond, oxygen, sulphur, imino or methylimino, with the proviso that if A¹ represents a direct bond, X² also represents a direct bond and vice versa;
X³-A³-X⁴-A⁴ represents an oxygen atom or a methyleneoxy group;
Y¹, Y² and Y³ independently represent hydroxy, alkoxy, mercapto, alkylthio, amino, mono- or di-alkylamino, alkyl, alkenyl, alkynyl, cycloalkyl, aryl or arylalkyl, wherein alkyl, alkoxy, alkenyl and alkynyl are linear or branched having 1-6 carbon atoms and may have substituents selected from hydroxy, methoxy, mercapto, methylthio, amino, methylamino, dimethylamino arid halogen, cycloalkyl has 3-8 carbon atoms, aryl is carbocyclic or heterocyclic having 5-10 ring atoms and cycloalkyl and aryl may have substituents selected from methyl, hydroxy, methoxy and halogen, whereby one of Y¹, Y² and Y³ may also represent a C₇-C₃₀ alkyl or alkenyl group, including a C₁₀-C₃₀ terpenoid group;
Z¹ and Z² independently represent oxygen or sulphur; and
n is 1 or 2;
or a pharmaceutically acceptable salt thereof;
with the proviso that the polyisoprenyl pyrophosphate analogue is not polyisoprenyl pyrophosphate itself;
together with a carrier adapted for protein isoprenylation inhibiting purposes.

9. Pharmaceutical composition according to claim 8, wherein Pren represents a farnesyl or geranyl-geranyl group; A¹ represents a direct bond or a methylene group; at least two of Y¹, Y² and Y³ are hydroxy or mercapto or a salt thereof; and n = 1.

10. Pharmaceutical composition according to claim 9, wherein the polyisoprenyl pyrophosphate analogue is farnesyl phosphonophosphonate.

## Patentansprüche

1. Verwendung eines Polyisoprenylpyrophosphat-Analogons zur Herstellung einer pharmazeutischen Zusammensetzung, die zur Inhibierung von Protein-Isoprenylierung geeignet ist, wobei das Polyisoprenylpyrophosphat-Analogon die Formel 1 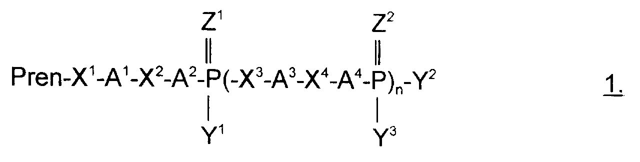 aufweist, worin:
Pren eine C₁₀-C₃₀-Terpenoid-Gruppe oder ein Derivat davon darstellt;
A¹, A², A³ und A⁴ unabhängig eine direkte Bindung oder eine C₁-C₄-Alkylen- oder -Alkenylengruppe, gegebenenfalls mit Substituenten, die aus Methyl, Hydroxy, Methoxy, Mercapto, Methylthio, Amino, Methylamino, Dimethylamino und Halogen ausgewählt sind, darstellen;
X¹, X², X³ und X⁴ unabhängig eine direkte Bindung, Sauerstoff, Schwefel, Imino oder Methylimino darstellen, mit der Maßgabe, daß, falls A¹ eine direkte Bindung darstellt, X² ebenfalls eine direkte Bindung darstellt und umgekehrt, und falls A³ eine direkte Bindung darstellt, X⁴ ebenfalls eine direkte Bindung darstellt und umgekehrt;
Y¹, Y² und Y³ unabhängig Hydroxy, Alkoxy, Mercapto, Alkylthio, Amino, Mono- oder Dialkylamino, Alkyl, Alkenyl, Alkinyl, Cycloalkyl, Aryl oder Arylalkyl darstellen, wobei Alkyl, Alkoxy, Alkenyl und Alkinyl linear oder verzweigt sind und 1-6 Kohlenstoffatome aufweisen und Substituenten aufweisen können, die aus Hydroxy, Methoxy, Mercapto, Methylthio, Amino, Methylamino, Dimethylamino und Halogen ausgewählt sind, Cycloalkyl 3-8 Kohlenstoffatome aufweist, Aryl carbocyclisch oder heterocyclisch mit 5-10 Ringatomen ist und Cycloalkyl und Aryl Substituenten aufweisen können, die aus Methyl, Hydroxy, Methoxy und Halogen ausgewählt sind, wobei eines von Y¹, Y² und Y³ auch eine C₇-C₃₀-Alkyl- oder -Alkenylgruppe, einschließlich einer C₁₀-C₃₀-Terpenoid-Gruppe, darstellen kann;
Z¹ und Z² unabhängig Sauerstoff oder Schwefel darstellen; und
n 1 oder 2 ist;
oder eines pharmazeutisch annehmbaren Salzes davon;
mit der Maßgabe, daß das Polyisoprenylpyrophosphat-Analogon nicht Polyisoprenylpyrophosphat selbst ist.

2. Verwendung nach Anspruch 1, worin die Gruppe X³-A³-X⁴-A⁴ ein Sauerstoffatom oder eine Methylenoxygruppe darstellt.

3. Verwendung nach Anspruch 1 oder 2, worin Pren eine Farnesyl- oder Geranyl-Geranyl-Gruppe darstellt.

4. Verwendung nach irgendeinem der Ansprüche 1-3, worin A¹ und A³ unabhängig eine direkte Bindung oder eine Methylengruppe darstellen und mindestens zwei von Y¹, Y² und Y³ Hydroxy oder Mercapto oder ein Salz davon sind; und n = 1.

5. Verwendung nach irgendeinem der Ansprüche 1-4, worin Y¹, Y² und Y³ Hydroxy oder Mercapto oder ein Salz davon sind.

6. Verwendung nach irgendeinem der vorhergehenden Ansprüche, worin die pharmazeutische Zusammensetzung zur Prophylaxe und/oder Behandlung von Karzinomen geeignet ist.

7. Verwendung nach irgendeinem der vorhergehenden Ansprüche, worin die pharmazeutische Zusammensetzung zur Inhibierung der Isoprenylierung eines ras-Proteins geeignet ist.

8. Pharmazeutische Zusammensetzung, die zur Inhibierung von Protein-Isoprenylierung geeignet ist, enthaltend ein Polyisoprenylpyrophosphat-Analogon der Formel 1: 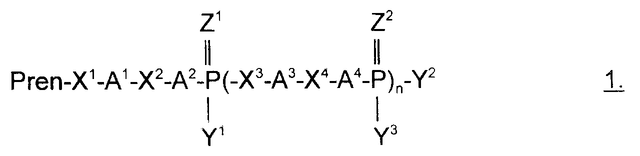 worin:
Pren eine C₁₀-C₃₀-Terpenoid-Gruppe oder ein Derivat davon darstellt;
A¹ und A² unabhängig eine direkte Bindung oder eine C₁-C₄-Alkylen- oder -Alkenylengruppe, gegebenenfalls mit Substituenten, die aus Methyl, Hydroxy, Methoxy, Mercapto, Methylthio, Amino, Methylamino, Dimethylamino und Halogen ausgewählt sind, darstellen;
X¹ und X² unabhängig eine direkte Bindung, Sauerstoff, Schwefel, Imino oder Methylimino darstellen, mit der Maßgabe, daß, falls A¹ eine direkte Bindung darstellt, X² ebenfalls eine direkte Bindung darstellt und umgekehrt;
X³-A³-X⁴-A⁴ ein Sauerstoffatom oder eine Methylenoxygruppe darstellt;
Y¹, Y² und Y³ unabhängig Hydroxy, Alkoxy, Mercapto, Alkylthio, Amino, Mono- oder Dialkylamino, Alkyl, Alkenyl, Alkinyl, Cycloalkyl, Aryl oder Arylalkyl darstellen, wobei Alkyl, Alkoxy, Alkenyl und Alkinyl linear oder verzweigt sind und 1-6 Kohlenstoffatome aufweisen und Substituenten aufweisen können, die aus Hydroxy, Methoxy, Mercapto, Methylthio, Amino, Methylamino, Dimethylamino und Halogen ausgewählt sind, Cycloalkyl 3-8 Kohlenstoffatome aufweist, Aryl carbocyclisch oder heterocyclisch mit 5-10 Ringatomen ist und Cycloalkyl und Aryl Substituenten aufweisen können, die aus Methyl, Hydroxy, Methoxy und Halogen ausgewählt sind, wobei eines von Y¹, Y² und Y³ auch eine C₇-C₃₀-Alkyl- oder -Alkenylgruppe, einschließlich einer C₁₀-C₃₀-Terpenoid-Gruppe, darstellen kann;
Z¹ und Z² unabhängig Sauerstoff oder Schwefel darstellen; und
n 1 oder 2 ist;
oder ein pharmazeutisch annehmbares Salz davon;
mit der Maßgabe, daß das Polyisoprenylpyrophosphat-Analogon nicht Polyisoprenylpyrophosphat selbst ist;
zusammen mit einem Träger, der für Zwecke der Inhibierung von Protein-Isoprenylierung adaptiert ist.

9. Pharmazeutische Zusammensetzung nach Anspruch 8, worin Pren eine Farnesyl- oder Geranyl-Geranyl-Gruppe darstellt; A¹ eine direkte Bindung oder eine Methylengruppe darstellt; mindestens zwei von Y¹, Y² und Y³ Hydroxy oder Mercapto oder ein Salz davon sind; und n = 1.

10. Pharmazeutische Zusammensetzung nach Anspruch 9, worin das Polyisoprenylpyrophosphat-Analogon Farnesylphosphonophosphonat ist.

## Revendications

1. Utilisation d'un analogue de pyrophosphate de polyisoprényle pour la préparation d'une composition pharmaceutique appropriée pour inhiber l'isoprénylation de protéines, l'analogue de pyrophosphate de polyisoprényle correspondant à la formule 1 : 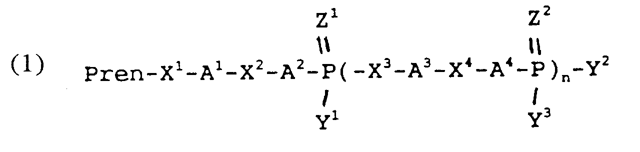
dans laquelle *Pren* représente un groupe terpénoïde en C₁₀₋₃₀ ou un dérivé de celui-ci,
A¹, A², A³ et A⁴ représentent indépendamment les uns des autres une liaison directe ou un groupe alcénylène ou alkylène en C₁₋₄, portant éventuellement des substituants choisis parmi les résidus méthyle, hydroxy, méthoxy, mercapto, méthylthio, amino, méthylamino, diméthylamino et halogéno;
X¹, X², X³ et X⁴ représentent indépendamment une liaison directe, un atome d'oxygène ou de soufre, un groupe imino ou méthylimino, avec la réserve que lorsque A¹ représente une liaison directe, X² représente également une liaison directe et vice versa, et lorsque A³ représente une liaison directe, X⁴ représente également une liaison directe et vice versa;
Y¹, Y² et Y³ représentent indépendamment un groupe hydroxy, alcoxy, mercapto, alkylthio, amino, mono- ou di-alkylamino, alkyle, alcényle, alcynyle, cycloalkyle, aryle ou arylalkyle, lesdits résidus alkyle, alcoxy, alcényle et alcynyle étant linéaires ou ramifiés, portant de 1 à 6 atomes de carbone et pouvant porter un ou plusieurs substituants choisis parmi les groupes hydroxy méthoxy, mercapto, méthylthio, amino, méthylamino, diméthylamino et halogéno, le groupe cycloalkyle comportant de 3 à 8 atomes de carbone, le groupe aryle étant un groupe carbocyclique ou hétérocyclique à 5 - 10 atomes formateur de cycle, et les groupes cycloalkyle et aryle pouvant porter des substituants choisis parmi les groupes méthyle, hydroxy, méthoxy et halogéno, un des groupes Y¹, Y² et Y³ pouvant ainsi également représenter un groupe alkyle ou alcényle en C₇₋₃₀, y compris un groupe terpénoïde en C₁₀₋₃₀ ;
Z¹ et Z² représentent indépendamment l'un de l'autre un atome d'oxygène ou de soufre ; et
n vaut 1 ou 2 ;
ou un sel pharmaceutiquement acceptable d'un tel composé, avec la réserve que l'analogue de pyrophosphate de polyisoprényle ne soit pas le pyrophosphate de polyisoprényle lui-même.

2. Utilisation selon la revendication 1, dans laquelle le groupe X³-A³-X⁴-A⁴ représente un atome d'oxygène ou un groupe méthylène-oxy.

3. Utilisation selon la revendication 1 ou 2, dans laquelle *Pren* représente un groupe farnésyle ou géranyl-géranyle.

4. Utilisation selon l'une quelconque des revendications 1 à 3, dans laquelle A¹ et A³ représentent indépendamment une liaison directe ou un groupe méthylène et au moins deux des symboles Y¹, Y² et Y³ représentent un groupe hydroxy ou mercapto, ou un sel de celui-ci, et n = 1.

5. Utilisation selon l'une quelconque des revendications 1 - 4, dans laquelle Y¹, Y² et Y³ représentent chacun un groupe hydroxy ou mercapto, ou un sel de celui-ci.

6. Utilisation selon l'une quelconque des revendications précédentes, dans laquelle la composition pharmaceutique convient pour la prophylaxie et/ou le traitement de carcinomes.

7. Utilisation selon l'une quelconque des revendications précédentes dans laquelle la composition pharmaceutique convient pour l'inhibition de l'isoprénylation de la protéine *ras*.

8. Composition pharmaceutique appropriée pour inhiber l'isoprénylation de protéines contenant un analogue de pyrophosphate de polyisoprényle de formule 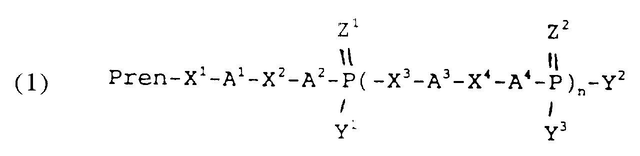 dans laquelle
*Pren* représente un groupe terpénoïde en C₁₀₋₃₀ ou un dérivé de celui-ci,
A¹ et A² représentent indépendamment une liaison directe ou un groupe alcénylène ou alkylène en C₁₋₄, portant éventuellement des substituants choisis parmi les résidus méthyle, hydroxy, méthoxy, mercapto, méthylthio, amino, méthylamino, diméthylamino et halogéno;
X¹ et X² représentent indépendamment une liaison directe, un atome d'oxygène ou de soufre, un groupe imino ou méthylimino, avec la réserve que lorsque A¹ représente une liaison directe, X² représente également une liaison directe et vice versa,
X³-A³-X⁴-A⁴ représente un atome d'oxygène ou un groupe méthylène-oxy ;
Y¹, Y² et Y³ représentent indépendamment un groupe hydroxy, alcoxy, mercapto, alkylthio, amino, mono- ou di-alkylamino, alkyle, alcényle, alcynyle, cycloalkyle, aryle ou arylalkyle, lesdits résidus alkyle, alcoxy, alcényle et alcynyle étant linéaires ou ramifiés, portant de 1 à 6 atomes de carbone et pouvant porter un ou plusieurs substituants choisis parmi les groupes hydroxy, méthoxy, mercapto, méthylthio, amino, méthylamino, diméthylamino et halogéno, le groupe cycloalkyle comportant de 3 à 8 atomes de carbone, le groupe aryle étant un groupe carbocyclique ou hétérocyclique à 5 - 10 atomes formateur de cycle, et les groupes cycloalkyle et aryle pouvant porter des substituants choisis parmi les groupes méthyle, hydroxy, méthoxy et halogéno, un des groupes Y¹, Y² et Y³ pouvant ainsi également représenter un groupe alkyle ou alcényle en C₇₋₃₀, y compris un groupe terpénoïde en C₁₀₋₃₀ ;
Z¹ et Z² représentent indépendamment l'un de l'autre un atome d'oxygène ou de soufre ; et
n vaut 1 ou 2 ;
ou un sel pharmaceutiquement acceptable d'un tel composé, avec la réserve que l'analogue de pyrophosphate de polyisoprényle n'est pas le pyrophosphate de polyisoprényle lui-même,
en combinaison avec un véhicule compatible avec l'inhibition d'isoprénylation de protéines.

9. Composition pharmaceutique selon la revendication 8, dans laquelle *Pren* représente un groupe farnésyle ou géranyl-géranyle, A¹ représente une liaison directe ou un groupe méthylène, au moins deux des résidus Y¹, Y² et Y³ représentent un résidu hydroxy ou mercapto, ou un sel de celui-ci, et n = 1.

10. Composition pharmaceutique selon la revendication 9, dans laquelle l'analogue de pyrophosphate de polyisoprényle est le phosphonophosphate de farnésyle.
